# EUROPÄISCHE PATENTSCHRIFT

(11) **EP 0 983 223 B1**
(45) Veröffentlichungstag und Bekanntmachung des Hinweises auf die Patenterteilung: **06.03.2002**
(21) Anmeldenummer: 98924186.4
(22) Anmeldetag: 23.04.1998
(51) Int. Cl.: C07C 45/00, C07C 49/80

(54) **VERFAHREN ZUR HERSTELLUNG VON ACETOPHENONEN, DIE AM AROMATISCHEN KERN MIT FLUORALKYL SUBSTITUIERT SIND**
METHOD FOR PREPARING ACETOPHENONES WITH A FLUORALKYL-SUBSTITUTED AROMATIC NUCLEUS
PROCEDE DE PREPARATION D'ACETOPHENONES SUBSTITUEES PAR FLUOROALKYLE AU NIVEAU DU NOYAU AROMATIQUE

(30) Priorität: 06.05.1997 DE 19719054
(43) Veröffentlichungstag der Anmeldung: 08.03.2000
(73) Patentinhaber: Bayer Aktiengesellschaft, 51368 Leverkusen (DE)
(72) Erfinder: KÜHNLE, Wulf, D-51061 Köln (DE); MARHOLD, Albrecht, D-51373 Leverkusen (DE); STEFFAN, Guido, D-51519 Odenthal (DE)
(86) Internationale Anmeldenummer: EP9802410
(87) Internationale Veröffentlichungsnummer: WO9850335

(56) Entgegenhaltungen:
- FR-A- 2 299 869
- "Method for preparation of acetophenone derivatives" RESEARCH DISCLOSURE, Nr. 402, Oktober 1997, Seite 706 XP000726840
- W.F. BEECH: "Preparation of aromatic aldehydes and ketones from diazonium salts" JOURNAL OF THE CHEMICAL SOCIETY., 1954, Seiten 1297-1302, XP002075827 LETCHWORTH GB

## Beschreibung

Die vorliegende Erfindung betrifft ein verbessertes Verfahren zur Herstellung von Acetophenonen, die am aromatischen Kern mit Fluoralkyl substituiert sind. Derartige Verbindungen sind wertvolle Zwischenprodukte zur Herstellung von antibakteriellen Wirkstoffen (s.DE-A 26 04 207).

Als Verfahren zur Herstellung von Trifluormethylacetophenon im technischen Maßstab ist bekannt, daß man aus Trifluormethylanilin und Natriumnitrit in Gegenwart von Salzsäure die entsprechende Diazoniumsalzlösung herstellt, diese mit Natriumacetat puffert, dann bei 5 bis 15°C zu einer Vorlage hinzufügt, die Wasser, Acetaldoxim, ein Kupfer(II)-salz, ein Reduktionsmittel (Natriumthiosulfat) und eine große Menge Natriumacetatpuffer enthält. Zur Aufarbeitung versetzt man mit Salzsäure, erhitzt zum Rückfluß, führt eine Wasserdampfdestillation durch, extrahiert und trocknet das Destillat, zieht das Extraktionsmittel ab und destilliert im Vakuum. Bezogen auf eingesetztes Fluormethylanilin erhält man so Trifluormethylacetophenon in einer Ausbeute von 33 % der Theorie (s. DE-A 26 04 207, Herstellungsbeispiel 1.4).

Nachteilig bei diesem Verfahren ist neben der niedrigen Ausbeute der Einsatz einer großen Menge an Hilfsstoffen, z.B. Puffersalzen, Reduktionsmitteln und Salzsäure, die die Aufarbeitung erschweren, zu einer erheblichen Salzfracht der Abwässer führen und nach ihrer Abtrennung großen Aufwand für eine umweltgerechte Entsorgung verursachen.

Andere bekannte Verfahren zur Herstellung von Trifluormethylacetophenon (s. beispielsweise Liebigs Ann. Chem. 717, 80 - 90 (1968), J.Am. Chem. Soc. 68, 736 (1946), J. Organomet. Chem. 489, (1-2), 137-143 (1995)) eignen sich nicht für die Durchführung im technischen Maßstab, weil sie teurer und schwierig zu handhabende Reagenzien und Apparaturen erfordern.

Es wurde nun ein Verfahren zur Herstellung von Acetophenonen, die am aromatischen Kern mit Fluoralkyl substituiert sind, aus Fluoralkylanilinen und Acetaldoxim gefunden, bei dem man aus einem Fluoralkylanilin eine entsprechende Diazoniumsalzlösung herstellt und diese mit Acetaldoxim in Gegenwart wenigstens einer Kupfer-Verbindung umsetzt, das dadurch gekennzeichnet ist, daß man keine Puffersalze und keine Reduktionsmittel zusetzt, die Umsetzung bei 20 bis 50°C und in Gegenwart von Halogenidionen durchführt und abschließend auf eine Temperatur im Bereich 70 bis 110°C erwärmt.

In das erfindungsgemäße Verfahren kann man z.B. Fluoralkylaniline der Formel (I) einsetzen 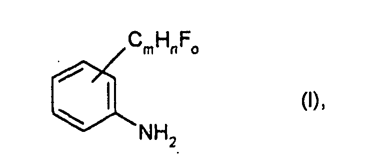 in der
- m: für 1, 2 oder 3,
- n: für (2m+1) - o und
- o: für (2m+1) - n stehen.

In Formel (I) stehen n vorzugsweise für Null und o vorzugsweise für (2m+1). Die CₘHₙFₒ-Gruppe befindet sich vorzugsweise in 3-Stellung zur Aminogruppe. Besonders bevorzugt wird 3-Trifluormethylanilin eingesetzt.

Die Herstellung der Diazoniumsalzlösung kann auf übliche Weise durchgeführt werden, beispielsweise durch Umsetzung mit Natriumnitrit in Gegenwart einer starken anorganischen Säure bei 0 bis -15°C. Es ist ein wesentliches Merkmal der vorliegenden Erfindung, daß man der fertigen Diazoniumsalzlösung keine Puffersalze, z.B. kein Natriumacetat, zusetzt.

Bezogen auf 1 mol eingesetztes Fluoralkylanilin kann man z.B. 1 bis 2 mol, vorzugsweise 1,2 bis 1,6 Acetaldoxim einsetzen.

Als Kupferverbindungen kommen beispielsweise Salze und Komplexverbindungen infrage, in denen das Kupfer in den Oxidationsstufen +1 oder +2 vorliegt. Beispiele für Salze sind Halogenide und Sulfate, Beispiele für Komplexverbindungen sind solche mit Hydroxylamin- oder Acetaldoxim-Liganden. Die Kupferverbindungen können wasserhaltig sein. Einzelbeispiele sind CuSO₄, CuSO₄ x 5 H₂O, CuCl, CuBr, CuCl₂, CuBr₂, Cu(O-N=CH-CH₃) und Cu(O-N=CH-CH₃)₂. Bezogen auf 1 mol eingesetztes Fluoralkylanilin kann man z.B. 0,04 bis 0,2 mol, vorzugsweise 0,045 bis 0,1 mol einer oder mehrerer Kupferverbindungen einsetzen.

Man kann das erfindungsgemäße Verfahren auf verschiedene Weise durchführen. Beispielsweise kann man ein Gemisch aus Acetaldoxim, Kupferverbindung(en) und wenig Wasser vorlegen und die Diazoniumsalzlösung dazudosieren. Man kann beispielsweise auch zunächst die Diazoniumsalzlösung herstellen, diese dann zu einer vorgelegten wäßrigen Lösung einer oder mehrerer Kupferverbindungen zudosieren und das entstandene Gemisch zu vorgelegtem Acetaldoxim zudosieren.

Es ist ein wesentliches Merkmal der vorliegenden Erfindung, daß man vor oder während der Umsetzung der Diazoniumsalzlösung mit Acetaldoxim keine Puffersalze (wie Natriumacetat) und keine Reduktionsmittel (wie Natriumthiosulfat) hinzufügt.

Die erfindungsgemäße Umsetzung wird vorzugsweise bei 25 bis 45°C durchgeführt.

Die erfindungsgemäß erforderliche Gegenwart von Halogenidionen während der Umsetzung kann im einfachsten Fall dadurch erreicht werden, daß man für die Herstellung der Diazoniumsalzlösung als starke anorganische Säure eine Halogenwasserstoffsäure verwendet, beispielsweise 25 bis 40 Gew.-%ige wäßrige Chlorwasserstoffsäure oder 25 bis 48 Gew.-%ige wäßrige Bromwasserstoffsäure. Falls man bei der Herstellung der Diazoniumsalzlösung eine andere starke anorganische Säure einsetzen will, kann man dies tun, muß dann jedoch Halogenidionen auf andere Weise zusetzen, beispielsweise in Form von Metallhalogenid, etwa in Form von NaCl oder KBr.

Bezogen auf 1 mol Kupferverbindungen liegen beispielsweise mindestens 1 mol, vorzugsweise mindestens 10 mol Halogenidionen im Reaktionsgemisch vor.

Es ist vorteilhaft, während des Dosierens der Diazoniumsalzlösung zum Acetaldoxim für eine intensive Durchmischung des Reaktionsgemisches zu sorgen, beispielsweise mittels eines Vibrationsmischers oder eines Dispergiergerätes der Art des Ultra-Turrax® .

Es ist weiterhin vorteilhaft, bei der Bereitung der miteinander umzusetzenden Komponenten und während der Umsetzung die Gegenwart von Sauerstoff weitgehend auszuschließen. Man kann hierzu beispielsweise in einer Stickstoffatmosphäre arbeiten, Stickstoff in die zu handhabenden Komponenten und/oder Reaktionsgemische einleiten oder ein leichtes Vakuum zur Entgasung anlegen.

Nach Beendigung der Zusammengabe der Diazoniumsalzlösung und der Acetaldoxim-Komponente kann man das Reaktionsgemisch gegebenenfalls noch einige Zeit, beispielsweise 15 Minuten bis 1 Stunde bei Temperaturen im Bereich 20 bis 50°C nachreagieren lassen.

Ein Versetzen mit (weiterer) Salzsäure vor der Aufarbeitung ist nicht erforderlich.

Wenn man das ausreagierte Reaktionsgemisch mittels Wasserdampfdestillation aufarbeitet, ist dem abschließenden Erwärmen auf 70 bis 110°C, vorzugsweise 80 bis 105°C, keine besondere Aufmerksamkeit zu widmen, weil man dann sowieso auf Temperaturen in diesem Bereich erwärmen muß. Wenn man andere Weise aufarbeiten will, z.B. durch Extraktion, ist vorher separat eine Erwärmung auf 70 bis 110°C durchzuführen, beispielsweise für 5 bis 30 Minuten.

Die nach der Aufarbeitung, z.B. durch Wasserdampfdestillation oder Extraktion, vorliegenden rohen Acetophenone, die am aromatischen Kern mit Fluoralkyl substituiert sind, kann man durch fraktionierte Destillation, z.B. über eine Kolonne, weiter reinigen.

Man kann so die gewünschten Produkte in Reinheiten von über 98 % und in Ausbeuten von 48 bis über 55 % der Theorie erhalten.

Wenn man ein Fluoralkylanilin der Formel (I) eingesetzt hat, dann erhält man das entsprechende Produkt der Formel 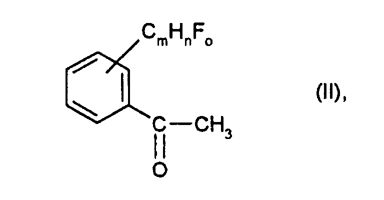 in der die verwendeten Symbole die bei Formel (I) angegebene Bedeutung haben.

Obwohl das erfindungsgemäße Verfahren im Gegensatz zum Stand der Technik ohne Zusatz von Puffersalzen und Reduktionsmittel auskommt und mit geringeren Mengen starker anorganischer Säuren und Kupferverbindungen durchgeführt werden kann, ist bei ihm die Ausbeute an Zielprodukten im allgemeinen um mindestens 50 % (relativ) erhöht. Das ist besonders überraschend und macht das erfindungsgemäße Verfahren wesentlich effizienter als die bekannten Verfahren. Die Abwassermengen und deren Salzgehalte sind wesentlich geringer und auch die Raumausbeute ist stark erhöht. Die erfindungsgemäß gegenüber dem Stand der Technik erhöhte Reaktionstemperatur ließ ein Ansteigen von Zersetzungsreaktionen erwarten, was jedoch nicht eintritt.

### Beispiele

### Beispiel 1

1290 g Wasser und 740 g Salzsäure (30 Gew.-%ig) wurden unter Stickstoff vorgelegt und auf -10 bis -5°C abgekühlt. Dann wurden bei unter -5°C 334 g 3-Aminobenzotrifluorid zudosiert (Gehalt 99,9 %). 149 g Natriumnitrit wurden in 690 g Wasser gelöst und im Verlauf von 2 Stunden bei unter 0°C zudosiert. Danach wurde 1 Stunde nachgerührt. Eine Vorlage aus 24 g Kupfersulfat-Hydrat, 73 g Wasser und 183 g Acetaldoxim wurden unter Stickstoff auf 28°C erwärmt und dann die zuvor hergestellte Diazoniumsalzlösung unter intensivem Rühren im Verlauf von 2 Stunden eindosiert. Man ließ noch 30 Minuten bei 30°C nachreagieren. Nach 1 Stunde erwärmte man auf 100°C und destillierte das Rohprodukt zusammen mit Wasser ab. Nach dem Abtrennen des Wassers aus dem Destillat wurde das Rohprodukt über eine Kolonne destilliert. Es wurden 202 g 3-Trifluormethylacetophenon erhalten (Gehalt nach GC 99,1 %). Das entspricht einer Ausbeute von 51,5 % der Theorie.

### Beispiel 2

Eine Diazoniumsalzlösung wurde wie in Beispiel 1 beschrieben hergestellt. Eine Vorlage aus 0,53 Gew.-Teilen (bezogen auf 3-Trifluormethylanilin) Acetaldoxim und 0,073 Gew.-Teilen (bezogen auf 3-Trifluormethylanilin) Kupfer-(II)-salz des Acetaldoxims wurde durch Durchleiten von Stickstoff vom Sauerstoff entgast und auf 40°C erwärmt. Innerhalb 1,5 Stunden wurde die Diazoniumsalzlösung zugetropft. Anschließend wurde auf 100°C erhitzt und das Rohprodukt zusammen mit Wasser abdestilliert. Nach der Phasentrennung und Reindestillation über eine Kolonne wurde 3-Trifluormethylacetophenon in einer Ausbeute von 56 % der Theorie erhalten.

### Beispiel 3

297 g Wasser und 246 g Bromwasserstoffsäure (48 %ig) wurden unter Stickstoff vorgelegt und 80 g 3-Trifluormethylanilin eingetragen. Die sich bildende Suspension wurde gerührt und auf -6°C abgekühlt. Nun wurden innerhalb 30 Minuten 37 g Natriumnitrit gelöst in 78 g Wasser zugetropft und 30 Minuten nachgerührt. Anschließend wurde die gebildete Lösung bei 30°C zu einer Vorlage aus 6 g Kupfersulfat-Pentahydrat gelöst in 26 g Wasser zudosiert. Diese Mischung wurde nun innerhalb von 1,5 Stunden auf eine Vorlage aus 45 g Acetaldoxim zugetropft und dabei die Temperatur so kontrolliert, daß sie 40°C nicht überstieg. Anschließend wurde auf 100°C erhitzt. Das Rohprodukt wurde zusammen mit Wasser abdestilliert. Nach Abtrennung des Wassers aus dem Destillat wurde das Rohprodukt über eine Kolonne destilliert. Es wurden 44 g (= 48 % der Theorie) 3-Trifluormethylacetophenon erhalten.

## Patentansprüche

1. Verfahren zur Herstellung von Acetophenonen, die am aromatischen Kern mit Fluoralkyl substituiert sind, aus Fluoralkylanilinen und Acetaldoxim, bei dem man aus einem Fluoralkylanilin eine entsprechende Diazoniumsalzlösung herstellt und diese mit Acetaldoxim in Gegenwart wenigstens einer Kupferverbindung umsetzt, **dadurch gekennzeichnet, daß** man keine Puffersalze und keine Reduktionsmittel zusetzt, die Umsetzung bei 20 bis 50°C und in Gegenwart von Halogenidionen durchführt und abschließend auf eine Temperatur im Bereich 70 bis 110°C erwärmt.

2. Verfahren nach Anspruch 1, **dadurch gekennzeichnet, daß** man Fluoralkylaniline der Formel (I) einsetzt 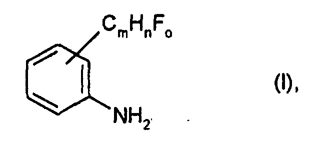 in der
m für 1, 2 oder 3,
n für (2m+1) - o und
o für (2m+1) - n stehen.

3. Verfahren nach Ansprüchen 1 und 2, **dadurch gekennzeichnet, daß** man die Diazoniumsalzlösung durch Umsetzen des Fluoralkylanilins mit Natriumnitrit in Gegenwart einer starken anorganischen Säure bei 0 bis -15°C durchführt.

4. Verfahren nach Ansprüchen 1 bis 3, **dadurch gekennzeichnet, daß** man auf 1 mol eingesetztes Fluoralkylanilin 1 bis 2 mol Acetaldoxim einsetzt.

5. Verfahren nach Ansprüchen 1 bis 4, **dadurch gekennzeichnet, daß** man als Kupferverbindung Salze und/oder Komplex-Verbindungen einsetzt, in denen das Kupfer in den Oxidationsstufen +1 oder +2 vorliegt und bezogen auf 1 mol eingesetztes Fluoralkylanilin 0,04 bis 0,2 mol einer oder mehrerer Kupferverbindungen einsetzt.

6. Verfahren nach Ansprüchen 1 bis 5, **dadurch gekennzeichnet, daß** man es durchführt, indem man ein Gemisch aus Acetaldoxim, Kupferverbindungen und wenig Wasser vorlegt und die Diazoniumlösung zudosiert oder die Diazoniumsalzlösung herstellt, diese zu einer vorgelegten wäßrigen Lösung einer oder mehrerer Kupferverbindungen zudosiert und das entstandene Gemisch zu vorgelegtem Acetaldoxim zudosiert.

7. Verfahren nach Ansprüchen 1 bis 6, **dadurch gekennzeichnet, daß** bezogen auf 1 mol Kupferverbindungen mindestens 1 mol Halogenidionen im Reaktionsgemisch vorliegen.

8. Verfahren nach Ansprüchen 1 bis 7, **dadurch gekennzeichnet, daß** man bei der Bereitung der miteinander umzusetzenden Komponenten während der Umsetzung die Gegenwart von Sauerstoff weitgehend ausschließt.

9. Verfahren nach Ansprüchen 1 bis 8, **dadurch gekennzeichnet, daß** man das ausreagierte Reaktionsgemisch durch Wasserdampfdestillation oder Extraktion und anschließende fraktionierte Destillation aufarbeitet.

## Claims

1. Process for preparing acetophenones which are substituted on the aromatic ring by fluoroalkyl from fluoroalkylanilines and acetaldoxime, in which from a fluoroalkylaniline a corresponding diazonium salt solution is prepared which is reacted with acetaldoxime in the presence of at least one copper compound, **characterized in that** no buffer salts and no reducing agents are added, the reaction is carried out at from 20 to 50°C and in the presence of halide ions and the mixture is subsequently heated to a temperature in the range from 70 to 110°C.

2. Process according to Claim 1, **characterized in that** fluoroalkylanilines of the formula (I) are employed 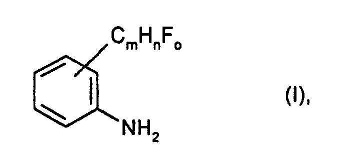 in which
m represents 1, 2 or 3,
n represents (2m+1) - o and
o represents (2m+1) - n.

3. Process according to Claims 1 and 2, **characterized in that** the diazonium salt solution is carried out by reacting the fluoroalkylaniline with sodium nitrite in the presence of a strong inorganic acid at from 0 to -15°C.

4. Process according to Claims 1 to 3, **characterized in that** 1 to 2 mol of acetaldoxime are employed per mole of fluoroalkylaniline used.

5. Process according to Claims 1 to 4, **characterized in that** the copper compounds used are salts and/or complex compounds in which the copper is present in the oxidation state +1 or +2 and from 0.04 to 0.2 mol of one or more copper compounds are employed, based on 1 mole of fluoroalkylaniline employed.

6. Process according to Claims 1 to 5, **characterized in that** the process is carried out by initially charging a mixture of acetaldoxime, copper compounds and a little water and the diazonium solution is metered in or the diazonium salt solution is prepared and metered into an aqueous solution of one or more copper compounds which had been charged initially, and the resulting mixture is metered into acetaldoxime which had been charged initially.

7. Process according to Claims 1 to 6, **characterized in that** at least 1 mole of halide ions is present in the reaction mixture, based on 1 mole of copper compounds.

8. Process according to Claims 1 to 7, **characterized in that** the presence of oxygen is substantially excluded during the preparation of the components to be reacted with one another during the reaction.

9. Process according to Claims 1 to 8, **characterized in that** the reaction mixture is, after the reaction has ended, worked up by steam distillation or extraction and subsequent fractional distillation.

## Revendications

1. Procédé pour la préparation d'acétophénones, lesquelles sont substituées au noyau aromatique par un groupe fluoroalkyle, à partir de fluoroalkylanilines et d'acétaldoxime, dans lequel on prépare une solution correspondante de sel de diazonium à partir d'une fluoroalkylaniline et on fait réagir celle-ci avec une acétaldoxime en présence d'au moins un composé du cuivre, **caractérisé en ce que** l'on n'ajoute pas de sel tampon ni d'agent réducteur, la réaction est réalisée à de 20 à 50°C et en présence d'ions halogénure et on chauffe ensuite à une température dans le domaine de 70 à 110°C.

2. Procédé selon la revendication 1, **caractérisé en ce que** l'on utilise des fluoroalkylanilines de la formule (I) 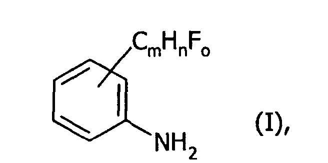 où
m représente 1, 2 ou 3,
n représente (2m+1)-o et
o représente (2m+1)-n.

3. Procédé selon les revendications 1 et 2, **caractérisé en ce que** l'on réalise la solution de sel de diazonium par réaction de la fluoroalkylaniline avec du nitrite de sodium en présence d'un acide fort inorganique à de 0 à -15°C.

4. Procédé selon les revendications 1 à 3, **caractérisé en ce que** l'on utilise pour 1 mol de fluoroalkylaniline utilisée de 1 à 2 mol d'acétaldoxime.

5. Procédé selon les revendications 1 à 4, **caractérisé en ce que** l'on utilise comme composé du cuivre des sels et/ou des composés complexes, dans lesquels le cuivre est présent dans les états d'oxydation +1 ou +2 et on utilise pour 1 mol de fluoroalkylaniline utilisée de 0,04 à 0,2 mol d'un ou plusieurs composés du cuivre.

6. Procédé selon les revendications 1 à 5, **caractérisé en ce qu'**il est réalisé **en ce que** l'on dispose d'un mélange constitué d'acétaldoxime, de composés du cuivre et de peu d'eau et que l'on ajoute la solution de diazonium ou que l'on prépare la solution de sel de diazonium, on ajoute celle-ci à une solution aqueuse préalablement disposée d'un ou plusieurs composés du cuivre et on ajoute le mélange produit à l'acétaldoxime préalablement disposée.

7. Procédé selon les revendications 1 à 6, **caractérisé en ce qu'**au moins 1 mol d'ions d'halogénure est présente dans le mélange réactionnel pour 1 mol de composés du cuivre.

8. Procédé selon les revendications 1 à 7, **caractérisé en ce que** l'on exclut largement pendant la réaction la présence d'oxygène dans la préparation des constituants à faire réagir les uns avec les autres.

9. Procédé selon les revendications 1 à 8, **caractérisé en ce que** l'on traite le mélange réactionnel ayant réagi par distillation à la vapeur d'eau ou par extraction et par distillation subséquente fractionnée.
